# EUROPEAN PATENT SPECIFICATION

(11) **EP 0 630 290 B1**
(45) Date of publication and mention of the grant of the patent: **23.12.1998**
(21) Application number: 93908319.2
(22) Date of filing: 11.03.1993
(51) Int. Cl.: B01J 29/06, C07C 2/12, C07C 5/52, B01J 31/02, C07C 6/12

(54) **PROCESS FOR PREPARING A SHAPE SELECTIVE CONVERSION CATALYST**
VERFAHREN ZUR HERSTELLUNG EINES FORMSELEKTIVEN UMWANDLUNGSKATALYSATORS
PROCEDE POUR LA PREPARATION D'UN CATALYSEUR DE CONVERSION A SELECTIVITE DE FORME

(30) Priority: 12.03.1992 US 850104; 12.03.1992 US 850105; 02.09.1992 US 939752
(43) Date of publication of application: 28.12.1994
(73) Proprietor: MOBIL OIL CORPORATION, Fairfax, Virginia 22037-0001 (US)
(72) Inventor: CHANG, Clarence, Dayton, Princeton, NJ 08540 (US); RODEWALD, Paul, Gerhard, Jr., Rocky Hill, NJ 08553 (US)
(74) Representative: Kador & Partner
(86) International application number: PCT/US93/02254
(87) International publication number: WO 93/17788

(56) References cited:
- FR-A- 2 566 389
- US-A- 3 682 996
- US-A- 4 090 981
- US-A- 4 127 616
- US-A- 4 465 886
- US-A- 4 477 583
- US-A- 4 950 835

## Description

The present invention is directed to a process for preparing a catalyst for shape selective conversions such as the conversion of toluene to para-xylene.

The term shape-selective catalysis describes unexpected catalytic selectivities in zeolites. The principles behind shape selective catalysis have been reviewed extensively, e.g. by N.Y. Chen, W.E. Garwood and F.G. Dwyer, "Shape Selective Catalysis in Industrial Applications," 36, Marcel Dekker, Inc. (1989). Within a zeolite pore, hydrocarbon conversion reactions such as paraffin isomerization, olefin skeletal or double bond isomerization, oligomerization and aromatic disproportionation, alkylation or transalkylation reactions are governed by constraints imposed by the channel size. Reactant selectivity occurs when a fraction of the feedstock is too large to enter the zeolite pores to react; while product selectivity occurs when some of the products cannot leave the zeolite channels. Product distributions can also be altered by transition state selectivity in which certain reactions cannot occur because the reaction transition state is too large to form within the zeolite pores or cages. Another type of selectivity results from configurational diffusion where the dimensions of the molecule approach that of the zeolite pore system. A small change in dimensions of the molecule or the zeolite pore can result in large diffusion changes leading to different product distributions. This type of shape selective catalysis is demonstrated, for example, in the selective disproportionation of toluene to p-xylene.

Para-xylene is a very valuable commercial product useful in the production of polyester fibers. The catalytic production of para-xylene has received much attention in the scientific community and various methods for increasing catalyst para-selectivity have been described.

The synthesis of para-xylene is typically performed by methylation of toluene in the presence of a suitable catalyst. Examples are the reaction of toluene with methanol as described by Chen et al., J. Amer. Chem. Sec. 1979, 101, 6783, and toluene disproportionation, as described by Pines in "The Chemistry of Catalytic Hydrocarbon Conversions", Academic Press, N.Y., 1981, p. 72. Such methods typically result in the production of a mixture including para-xylene, ortho-xylene, and meta-xylene. Depending upon the para-selectivity of the catalyst and the reaction conditions, different percentages of para-xylene are obtained. The yield, i.e., the amount of feedstock actually converted to xylene, is also affected by the catalyst and the reaction conditions.

The equilibrium reaction for the disproportionation of toluene to xylene and benzene proceeds as follows: 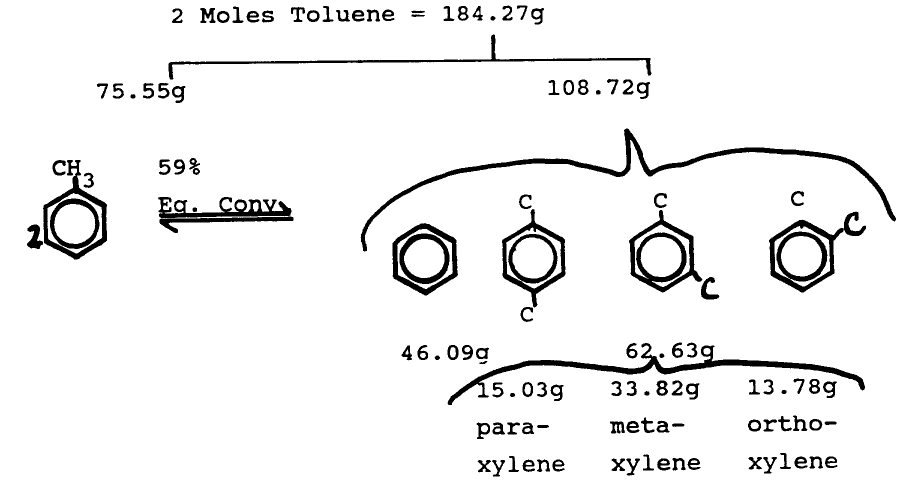 $\begin{matrix}\begin{matrix}\text{p-Xylene Yield=SelectivityxConversion=} \frac{\text{15.03}}{\text{108.72}} \text{x0.59=8.2%} \\ \text{p-Xylene purity=100x} \frac{\text{15.03}}{\text{62.63}} \text{=24%}\end{matrix}\end{matrix}$

One known method for increasing para-selectivity of a zeolite catalyst is to modify the catalyst by treatment with a "selectivating agent". Modification methods have been suggested wherein the catalyst is modified by treatment prior to use to provide a silica coating. For example, U.S. Patents 4,477,583 and 4,127,616 disclose methods wherein a catalyst is contacted at ambient conditions with a modifying compound such as phenylmethyl silicone in a hydrocarbon solvent or an agueous emulsion, followed by calcination. Such modification procedures have been successful in obtaining para-selectivity of up to about 90% but only at the expense of commercially unacceptable toluene conversions of about 10%, resulting in a yield of not greater than about 9%, i.e. 10% x 90%. Such processes also produce significant quantities of ortho-xylene and meta-xylene thereby necessitating expensive separation processes, such as fractional crystallization and adsorptive separation in order to separate the para-xylene from the other isomers. The other xylene isomers are customarily recycled, thereby requiring xylene isomerization units for additional conversion of the recycled xylene isomers into an equilibrium xylene mixture comprising para-xylene.

Those skilled in the art appreciate that the expense of the separation process is proportional to the degree of separation required. Therefore, significant cost savings are achieved by increasing selectivity to the para-isomer while maintaining commercially acceptable conversion levels.

Other conversion processes which benefit from the shape selectivity of the catalyst employed include dewaxing of hydrocarbon feedstocks; isomerization of alkylaromatics; oligomerization of olefins to form gasoline, distillate, lube oils or chemicals; alkylation of aromatics; conversion of oxygenates to hydrocarbons; rearrangement of oxygenates; and conversion of light paraffins and olefins to aromatics.

It is, therefore, an object of the present invention to provide an improved shape selective conversion catalyst.

Accordingly, the invention resides in a process for producing a shape selective conversion catalyst comprising converting toluene to xylene by contacting toluene with a catalyst comprising a molecular sieve having a Constraint Index of 1-30 at a temperature of 100-600°C, a pressure of 100 to 14000 kPa (atmospheric to 2000 psig), a WHSV of 0.1 to 100, and a hydrogen to hydrocarbon molar ratio of 0 to 10, characterized in that the toluene is in the form of a vapour phase mixture which comprises at least 80% by weight of toluene and at least 0.1% by weight of a selectivating agent.

The molecular sieve catalyst used in the processes of the present invention preferably has an initial constraint index of 1-20, more preferably 1-12, and preferably comprises an intermediate pore-size zeolite such as ZSM-5, ZSM-11, ZSM-22, ZSM-23, or ZSM-35, most preferably ZSM-5. The catalyst preferably has an alpha value greater than 100, for example 150-2000, and a silica-alumina ratio less than 100, preferably 20-80. The Alpha Value of the catalyst may be increased by treating the catalyst with nitric acid or by mild steaming as discussed in US Patent 4326994.

The Alpha Value is an approximate indication of the catalytic cracking activity of the catalyst compared to a standard catalyst and it gives the relative rate constant (rate of normal hexane conversion per volume of catalyst per unit time). It is based on the activity of an amorphous silica-alumina cracking catalyst taken as a Alpha of 1 (Rate Constant = 0.016 sec⁻¹). The Alpha Test is described in US Patent 3354078 and in The Journal of Catalysis, Vol. 4, pp. 522-529 (August 1965): Vol. 6, p. 278 (1966); and Vol. 61, p. 395 (1980). It is noted that intrinsic rate constants for many acid-catalyzed reactions are proportional to the Alpha Value for a particular crystalline silicate catalyst (see "The Active Site of Acidic Aluminosilicate Catalysts," Nature, Vol. 309, No. 5959, pp. 589-591, 14 June 1984). The experimental conditions of the test used herein include a constant temperature of 538°C and a variable flow rate as described in detail in the Journal of Catalysis, Vol. 61, p. 395. Constraint index and the manner by which it is determined are described in U.S. Patent No. 4,016,218.

The molecular sieve catalyst employed in the processes of the invention may be used in combination with a support or binder material such as, for example, a porous inorganic oxide support or a clay binder. The preferred binder is primarily silica. Other non-limiting examples of binder materials include alumina, zirconia, magnesia, thoria, titania, boria and combinations thereof, generally in the form of dried inorganic oxide gels or gelatinous precipitates. Suitable clay materials include, by way of example, bentonite and kieselguhr. The relative proportion of suitable crystalline molecular sieve to the total composition of catalyst and binder or support may be 30-90 percent by weight and is preferably 50-80 percent by weight of the composition. The composition may be in the form of an extrudate, beads, or fluidizable microspheres.

The molecular sieve catalyst is provided with the required shape selectivity by treatment (hereinafter referred to as "trim-selectivation") with a mixture of a selectivating agent, hydrogen and toluene under conditions sufficient to disproportionate the toluene to xylene. The selectivating agent is fed in an amount of preferably 0.1-50%, more preferably 0.1-20%, by weight of the toluene. The trim-selectivating agent is preferably a volatile organosilicon compound and the reaction conditions preferably conveniently comprise a temperature of 300°C to 500°C; a pressure of 200 to 5600 kPa (15 to 800 psig); a mole ratio of hydrogen to hydrocarbons of 1 to 4 and a weight hourly space velocity (WHSV) of 0.1 to 10. The trim-selectivation process preferably occurs in situ ink the reaction vessel used for the conversion process to be catalysed and preferably lasts for 50-300 hours, most preferably less than 170 hours. Upon thermolysis, a siliceous coating is deposited on the zeolite surface, reducing or eliminating surface activity and enhancing shape-selectivity.

Preferably, prior to trim selectivation, the molecular sieve catalyst is subjected to ex-situ pre-treatment with a selectivating agent (hereinafter referred to as "pre-selectivation") in the form of a silicon-containing compound. For pre-selectivation the silicon compound is deposited on the external surface of the catalyst by any suitable method. For example, the silicon compound may be dissolved in a solvent, mixed with the catalyst, and then dried. The silicon compound employed may be in the form of a solution, a liquid or a gas under the conditions of contact with a zeolite. Examples of methods of depositing silicon on the surface of the zeolite are found in U.S. Patents 4,090,981, 4,127,616, 4,465,886 and 4,477,583.

Following deposition of the silicon-containing compound in pre-selectivation, the catalyst is preferably calcined. For example, the catalyst may be calcined in an oxygen-containing atmosphere, preferably air, at a rate of 0.2° to 5°C./minute to a temperature greater 300° C. but below a temperature at which the crystallinity of the zeolite is adversely affected. Generally, such temperature will be below 600°C. Preferably the temperature of calcination is within the approximate range of 350° to 550°C. The product is maintained at the calcination temperature usually for 1 to 24 hours.

Subjecting the catalyst to pre-selectivation is found to reduce the time taken and the amount of selectivating agent required to trim-selectivate the catalyst.

The silicon compounds used for the pre-selectivation and/or the trim-selectivation may comprise a polysiloxane including silicones, a siloxane, and a silane including disilanes and alkoxysilanes.

Silicone compounds which can be used in the present invention can be characterized by the general formula: 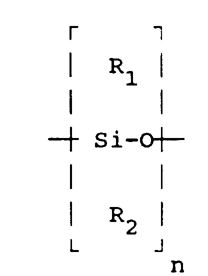 where R₁ is hydrogen, fluorine, hydroxy, alkyl, aralkyl, alkaryl or fluoro-alkyl. The hydrocarbon substituents generally contain from 1 to 10 carbon atoms and preferably are methyl or ethyl groups. R₂ is selected from the same group as R₁, and n is an integer of at least 2 and generally in the range of 2 to 1000. The molecular weight of the silicone compound employed is generally between 80 and 20,000 and preferably 150 to 10,000. Representative silicone compounds include dimethylsilicone, diethylsilicone, phenylmethylsilicone, methylhydrogensilicone, ethylhydrogensilicone, phenylhydrogensilicone, methylethylsilicone, phenylethylsilicone, diphenylsilicone, methyltrifluoropropylsilicone, ethyltrifluoro- propysilicone, tetrachlorophenylmethyl silicone, tetrachlorophenylethyl silicone, tetrachlorophenyl- hydrogen silicone, tetrachlorophenylphenyl silicone, methylvinylsilicone and ethylvinylsilicone. The silicone compound need not be linear but may be cyclic as for example hexamethylcyclotrisiloxane, octamethyl- cyclotetrasiloxane, hexaphenylcyclotrisiloxane and octaphenylcyclotetrasiloxane. Mixtures of these compounds may also be used as well as silicones with other functional groups.

Useful siloxanes or polysiloxanes include as non-limiting examples hexamethylcyclotrisiloxane, octamethylcyclotetrasiloxane, decamethylcyclopentasiloxane, hexamethyldisiloxane, octamethytrisiloxane, decamethyltetrasiloxane, hexaethylcyclotrisiloxane, octaethylcyclotetrasiloxane, hexaphenylcyclotrisiloxane and octaphenyl-cyclotetrasiloxane.

Useful silanes, disilanes or alkoxysilanes include organic substituted silanes having the general formula: 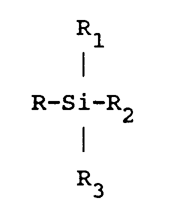 wherein R is a reactive group such as hydrogen, alkoxy, halogen, carboxy, amino, acetamide, trialkylsilyl. R₁, R₂ and R₃ can be the same as R or an organic radical which may include alkyl of from 1 to 40 carbon atoms, alkyl or aryl carboxylic acid wherein the organic portion of the alkyl contains 1 to 30 carbon atoms and the aryl group contains 6 to 24 carbon atoms, aryl groups of 6 to 24 carbons which may be further substituted, alkylaryl and arylalkyl groups containing 7 to 30 carbon atoms. Preferably, the alkyl group of an alkyl silane is between 1 and 4 carbon atoms in chain length. Mixtures may also be used.

The silanes or disilanes include, as non-limiting examples, dimethylphenylsilane, phenytrimethylsilane, triethylsilane and hexamethyldisilane. Useful alkoxysilanes are those with at least one silicon-hydrogen bond.

While not wishing to be bound by theory, it is believed that the advantages of the present invention are obtained by rendering acid sites on the external surfaces of the catalyst substantially inaccessible to reactants while increasing catalyst tortuosity. Acid sites existing on the external surface of the catalyst are believed to isomerize the para-xylene exiting the catalyst pores back to an equilibrium level with the other two isomers thereby reducing the amount of para-xylene in the xylenes to only about 24%. By reducing the availability of these acid sites to the para-xylene exiting the pores of the catalyst, the relatively high level of para-xylene can be maintained. It is believed that the selectivating agents of the present invention block or otherwise render these external acid sites unavailable to the para-xylene by chemically modifying said sites.

Preferably, the kinetic diameter of the selectivating agent is larger than the zeolite pore diameter, in order to avoid reducing the internal activity of the catalyst.

It is also believed that the presence of hydrogen in the reaction zone is important when a silicone compound is used as the trim-selectivating agent.

The selectivated catalyst made by the process of of the present invention is suitable for use in a variety of shape selective hydrocarbon conversion processes including as non-limiting examples, cracking hydrocarbons with reaction conditions including a temperature of 300°C to 700°C, a pressure of 10 to 3000 kPa (0.1 to 30 atmospheres) and a weight hourly space velocity of 0.1 to 20; dehydrogenating hydrocarbon compounds with reaction conditions including a temperature of 300°C to 700°C, a pressure of 10 to 1000 kPa (0.1 to 10 atmospheres) and weight hourly space velocity of 0.1 to 20; converting paraffins to aromatics with reaction conditions including a temperature of 300°C to 700°C, a pressure of 10 to 6000 kPa (0.1 to 60 atmospheres), a weight hourly space velocity of 0.5 to 400 and a hydrogen/hydrocarbon mole ratio of 0 to 20; converting olefins to aromatics, e.g. benzene, toluene and xylene, with reaction conditions including a temperature of 100°C to 700°C, a pressure of 10 to 6000 kPa (0.1 to 60 atmospheres), a weight hourly space velocity of 0.5 to 400 and a hydrogen/hydrocarbon mole ratio of 0 to 20; converting alcohols, e.g. methanol, or ethers, e.g. dimethylether, or mixtures thereof to hydrocarbons including olefins and/or aromatics with reaction conditions including a temperature of 275°C to 600°C, a pressure of 50 to 5000 kPa (0.5 to 50 atmospheres) and a liquid hourly space velocity of 0.5 to 100; isomerizing xylene feedstock components with reaction conditions including a temperature of 230°C to 510°C, a pressure of 300 to 3500 kPa (3 to 35 atmospheres), a weight hourly space velocity of 0.1 to 200 and a hydrogen/hydrocarbon mole ratio of 0 to 100; disproportionating toluene with reaction conditions including a temperature of 200°C to 760°C, a pressure of 100 to 6000 kPa (atmospheric to 60 atmospheres) and a weight hourly space velocity of 0.08 to 20.

The catalyst prepared by the process of of the invention is particularly useful in the disproportionation of toluene to para-xylene. For example, a para-xylene purity of greater than 90%, preferably at least 95%, based on all C₈ products can be attained with a toluene conversion of greater than 15%, preferably at least 20%, and most preferably at least 25%. Moreover, the ortho-xylene isomer can be reduced to not more than about 0.5% of the total xylene content while the meta-xylene isomer can be reduced to less than about 5% of the total xylene content. In addition, when the reaction system is properly treated, such as by deposition of platinum on the molecular sieve, the presence of ethylbenzene can be significantly reduced, e.g., to less than about 2% of the C₈ product.

Operating conditions for the toluene disproportionation process of the present invention include a temperature of 350 - 540°C, preferably greater than 400°C, a pressure of 100 to 35000 kPa (atmospheric to 5000 psig), preferably 800 to 7000 kPa (100 to 1000 psig), a WHSV of 0.1-20, preferably 2-4, and a hydrogen to hydrocarbon mole ratio of 0.1-20, preferably 2-4. This process may be conducted in either fixed- or fluid-bed mode with attendant benefits of either operation readily obtainable.

The effluent is separated and distilled to remove the desired product, i.e., para-xylene, plus other by-products. The unreacted reactant, i.e. toluene, is preferably recycled for further reaction. The benzene is a valuable co-product.

In accordance with a preferred embodiment of the present invention, when used in the disproportionation of toluene, the catalyst is further modified in order to reduce the amount of undesirable by-products, particularly ethylbenzene. The state of the art is such that the reactor effluent from toluene disproportionation typically contains about 0.5% ethylbenzene by-product. Upon distillation of the reaction products, the level of ethylbenzene in the C₈ fraction often increases to 3-4 percent. This level of ethylbenzene is unacceptable for polymer grade p-xylene since ethylbenzene in the C₈ product, if not removed, degrades the quality of fibers ultimately produced from the p-xylene product. Consequently, ethylbenzene content must be kept low. The specification for ethylbenzene in the C₈ product has been determined by industry to be less than 0.3%. The ethylbenzene can be substantially removed by isomerization or by superfractionation processes. Removal of the ethylbenzene by conventional isomerization would be impractical with the present invention since the xylene stream, which comprises greater than 90% para-xylene, would be concurrently isomerized to equilibrium xylenes reducing the amount of para-xylene in this xylene stream to about 24%. Moreover, it is known that the alternative procedure of removing the ethylbenzene by superfractionation is extremely expensive.

In order to avoid the need for downstream ethylbenzene removal, the level of ethylbenzene by-product is advantageously reduced by incorporating a hydrogenation-dehydrogenation function in the catalyst via addition of a metal compound such as platinum. While platinum is the preferred metal, other metals such as palladium, nickel, copper, cobalt, molybdenum, rhodium, ruthenium, silver, gold, mercury, osmium, iron, zinc, cadmium, and mixtures thereof may be utilized. The metal may be added by cation exchange, in amounts of 0.01 - 2%, typically about 0.5%. The metal must be able to enter the pores of the catalyst in order to survive a subsequent calcination step. For example, a platinum modified catalyst can be prepared by first adding to the catalyst a solution of ammonium nitrate in order to convert the catalyst to the ammonium form, and subsequently, an aqueous solution of tetraamine platinum(II) nitrate to increase activity. The catalyst can then be filtered, washed with water and calcined at temperatures of 250° to 500°C.

The invention will now be more particularly described with reference to the Examples and the accompanying drawings, in which:
Figure 1 is a graph comparing xylene para-selectivity and toluene conversion over a silicone trim-selectivated ZSM-5 catalyst in the presence of hydrogen (Example 1) or nitrogen as a function of stream time,
Figure 2 is a graph similar to Figure 1 and provides results of a hydrogen co-feed at the slightly lower temperature used in Example 2,
Figure 3 is also a graph similar to Figure 1 and shows results obtained in the absence of a hydrogen co-feed (Example 3),
Figure 4 is a graph showing para-xylene and toluene conversion as a function of time on stream.
Figure 5 depicts para-selectivity and conversion rates for a zeolite which has been pre-selectivated with 10% SiO₂, and
Figure 6 provides para-selectivity and conversion rates for a zeolite which has been pre-selectivated with 5% SiO₂.

### EXAMPLE 1

Toluene disproportionation was carried out in a fixed-bed reactor using 2 grams of a silica bound HZSM-5 catalyst having a silica/alumina ratio of 26, a crystal size of 0.1 micron, an Alpha Value of 731. The feed to the reactor was toluene containing 1% silicone compound having a phenylmethyl silicone to dimethyl silicone ratio of 1:1. Operating conditions were 4.0 WHSV, 480°C, 3550 kPa (500 psig), and a hydrogen/hydrocarbon ratio of 2. Table 1 summarizes toluene conversion and para-xylene selectivity as a function of time on stream during and after trim-selectivation.

**TABLE 1**

| Time on Stream, hr | Conversion, wt% | para-Xylene in Xylenes, wt% |
|---|---|---|
| 1 | 56 | 22 |
| 6 | 57 | 21 |
| 22 | 51 | 24 |
| 46 | 42 | 39 |
| 98 | 36 | 70 |
| 143 | 28 | 86 |
| 170 | 25 | 89 |
| 174* | 25 | 91 |
| 342* | 25 | 91 |

| | | |
|---|---|---|
| * Silicone co-feed discontinued. | | |

It is noteworthy that the silicone trim-selectivation substantially increased para-xylene selectivity from an initial 22% to over 90%. At 174 hours on stream the feed was changed to 100% toluene, i.e., the silicone co-feed was discontinued. Over the following one week test period, toluene conversion remained constant at 25% and para-xylene selectivity remained constant at 91%.

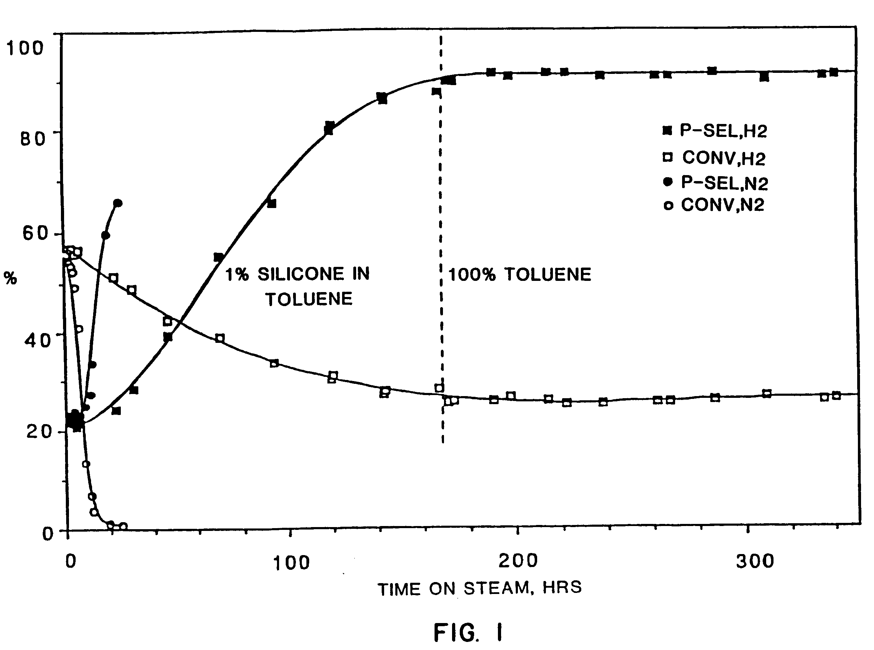

The above results are illustrated by Figure 1, which also includes the results of conducting the selectivation in the presence of nitrogen, rather than hydrogen. In the presence of nitrogen, the catalyst deactivated rapidly and conversion quickly approached zero.

### EXAMPLE 2

The toluene disproportionation process of Example 1 was repeated at 4.0 WHSV, 446°C, 3550 kPa (500 psig), and a hydrogen/hydrocarbon ratio = 2. Table 2 summarizes toluene conversion and para-xylene selectivity as a function of time on stream.

**TABLE 2**

| Time on Stream, hr | Conversion, wt% | p-Xylene in Xylenes, wt% |
|---|---|---|
| 1 | 44 | 29 |
| 25 | 42 | 34 |
| 47 | 37 | 58 |
| 94 | 31 | 86 |
| 143 | 29 | 93 |
| 176 | 27 | 96 |
| 199 | 26 | 97 |
| 223 | 25 | 97 |
| 239* | 25 | 97 |

| | | |
|---|---|---|
| * Silicone co-feed discontinued. | | |

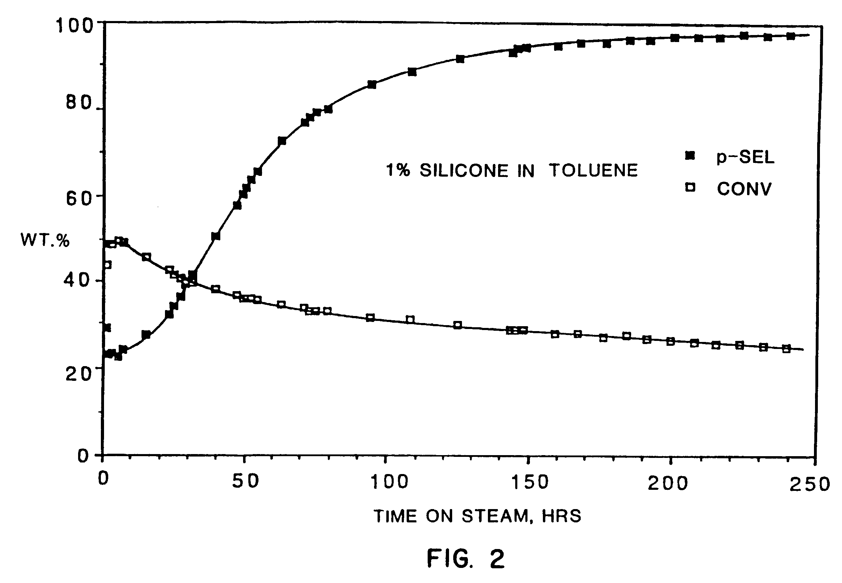

Silicone trim-selectivation increased para-xylene selectivity from 24% (thermodynamic value) to a high 97% at 25% toluene conversion. When the silicone co-feed was discontinued, the para-xylene selectivity and toluene conversion were unchanged at 97% and 25%, respectively. The results are illustrated by Figure 2.

### EXAMPLE 3

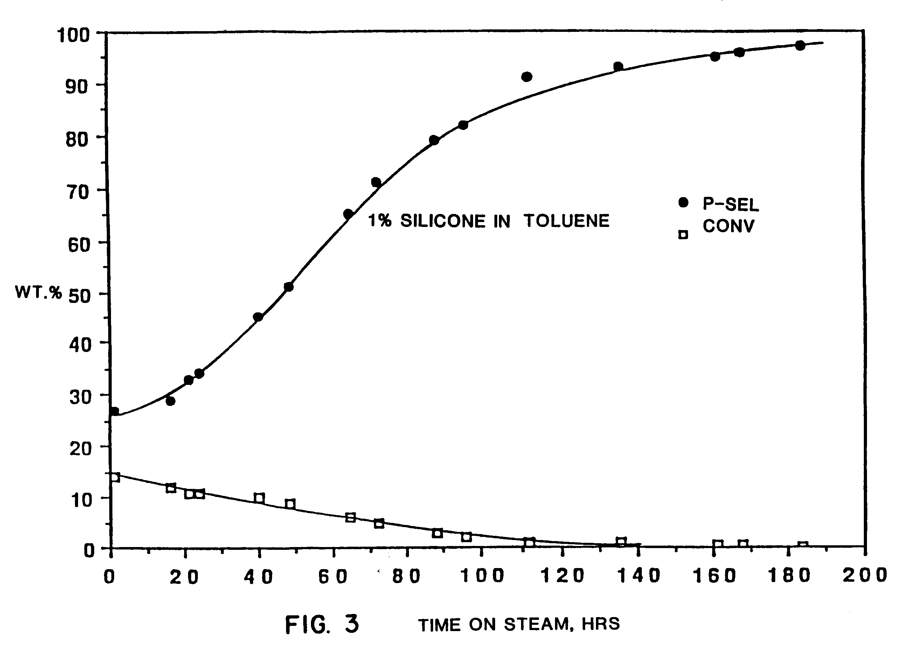

The toluene disproportionation process of Example 1 was repeated at 4.0 WHSV, 420°C, 100 kPa (0 psig), and hydrogen/hydrocarbon ratio = 0. Table 3 and Figure 3 summarize toluene conversion and para-xylene selectivity as a function of time on stream. Note that the conversion drops to essentially zero at 184 hours on stream in contrast to operation in the presence of hydrogen where at 184 hours on stream conversion has stabilized at 25%.

**TABLE 3**

| Time on Stream, hr | Conversion, wt% | para-Xylene in Xylenes, wt% |
|---|---|---|
| 1 | 14 | 27 |
| 48 | 8 | 51 |
| 96 | 2 | 82 |
| 136 | 1 | 93 |
| 184 | 0.1 | 97 |

### EXAMPLE 4

Toluene disproportionation over SiO₂-HZSM-5 was carried out using 1% octamethylcyclotetrasiloxane in a toluene feed as the trim-selectivating agent. Operating conditions were 446°C, 3550 kPa (500 psig), 4.0 WHSV, and H₂/HC=2. Table 4 summarizes the results.

**TABLE 4**

| Time on Stream, Hrs. | p-Xylene/Xylenes, wt% | Toluene Conversion, wt% |
|---|---|---|
| 0 | 25 | 40 |
| 24 | 88 | 23 |
| 46 | 95 | 18 |
| 71 | 98 | 15 |

### EXAMPLE 5

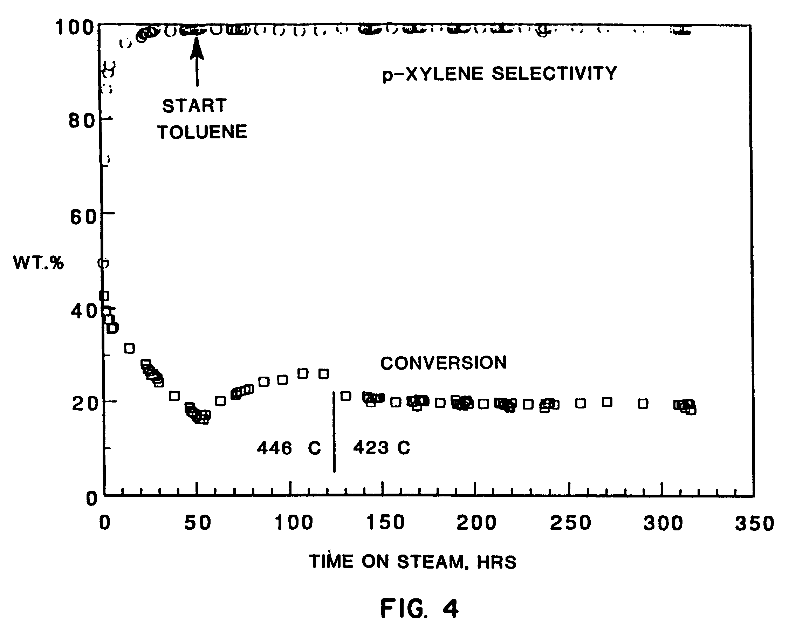

Toluene disproportionation with trim-selectivation as in Example 4 was carried out using hexamethyldisiloxane (HMDS). Table 5 and Figure 4 summarize the results.

**TABLE 5**

| Time on Streams, hrs. | p-Xylene in Xylenes, wt% | Toluene Conversion, wt% |
|---|---|---|
| 1 | 28 | 47 |
| 2 | 56 | 42 |
| 4 | 80 | 37 |
| 14 | 95 | 33 |
| 24 | 98 | 28 |
| 47 | 99 | 20 |
| 54 | 99 | 18 |

Figure 4 illustrates the high p-xylene selectivity and toluene conversion over 350 hours on stream. The toluene conversion remained at about 18 - 20% with a p-xylene selectivity of 99% for an extended period of time. HMDS was discontinued after about 50 hours.

### EXAMPLES 6-14

The process of Example 4 was repeated out with trim-selectivation using the siloxanes listed in Table 6. Operating conditions were 446°C, 3550 kPa (500 psig), 4.0 WHSV and H₂/HC = 2. The results after 24 hours are shown in Table 6.

**TABLE 6**

| Ex. | Siloxanes | p-Xylene/Xylenes, wt.% | Toluene Conversion wt.% |
|---|---|---|---|
| 6 | Methylhydrocyclosiloxanes | 89 | 13 |
| 7 | Hexamethylcyclotrisiloxane | 84^{a} | 20 |
| 8 | 1,3,5-Trimethyl-1,3,5-triphenylcyclotrisiloxane | 85 | 31 |
| 9 | Octamethylcyclotetrasiloxane | 88 | 23 |
| 10 | Decamethylcyclopentasiloxane | 90 | 28 |
| 11 | Decamethyltetrasiloxane | 98 | 24 |
| 12 | Hexamethyldisiloxane | 98 | 24 |
| 13 | 1,1,3,3,5,5-Hexamethyltrisiloxane | 96 | 14 |
| 14 | Octamethyltrisiloxane (after 41 hrs) | 81^{a} | 20 |

| | | | |
|---|---|---|---|
| ^{a} - continuation of the selectivation beyond the cited time periods brings the p-xylene selectivity to above 90% with toluene conversion of at least 15%. | | | |

### EXAMPLES 15-19

Trim-selectivations as in Examples 4 and 5 were carried out with the silanes listed in Table 7. Operating conditions were 446°C, 3550 kPa (500 psig), 4.0 WHSV and H₂/HC = 2. The results after 24 hours are shown in Table 7.

**TABLE 7**

| Ex. | Siloxanes | p-Xylene/Xylenes, wt.% | Toluene Conversion wt.% |
|---|---|---|---|
| 15 | Diphenylsilane | 96 | 15 |
| 16 | Dimethylphenylsilane | 97 | 19 |
| 17 | Phenyltrimethylsilane | 86^{a} | 19 |
| 18 | Triethylsilane | 85^{a} | 21 |
| 19 | Hexamethyldisilane | 95 | 23 |

| | | | |
|---|---|---|---|
| ^{a} - continuation of the selectivation beyond 24 hours brings the p-xylene selectivity to above 90%. | | | |

### EXAMPLES 20-24

For comparison purposes, the compounds listed in Table 8 were tested as in Examples 6-19 with results shown in Table 8.

**TABLE 8**

| Ex. | Sioxanes | p-Xylene/Xylenes, wt.% | Toluene Conversion wt.% |
|---|---|---|---|
| 20 | Hexaphenylcyclotrisiloxane | 43 | 29 |
| 21 | Octaphenylcyclotetrasiloxane | 66 | 28 |
| 22 | Tetra-(n-butyl)-orthosilicate | 36 | 2 |
| 23 | Tetra-ethyl-orthosilicate | 38 | 3 |
| 24 | Tetra-(2-ethylhexyl)-orthosilicate | 33 | 1 |

### EXAMPLE 25

A silica pre-selectivated ZSM-5 catalyst was prepared by adding 5.00 g HZSM-5 to 1.26 g phenylmethylpolysiloxane dissolved in 40 cc hexane. The solvent was distilled and the catalyst was air calcined at 1°C/min to 538°C, then 6 hours at 538°C. The pre-selectivated catalyst contained a nominal 10% added silica.

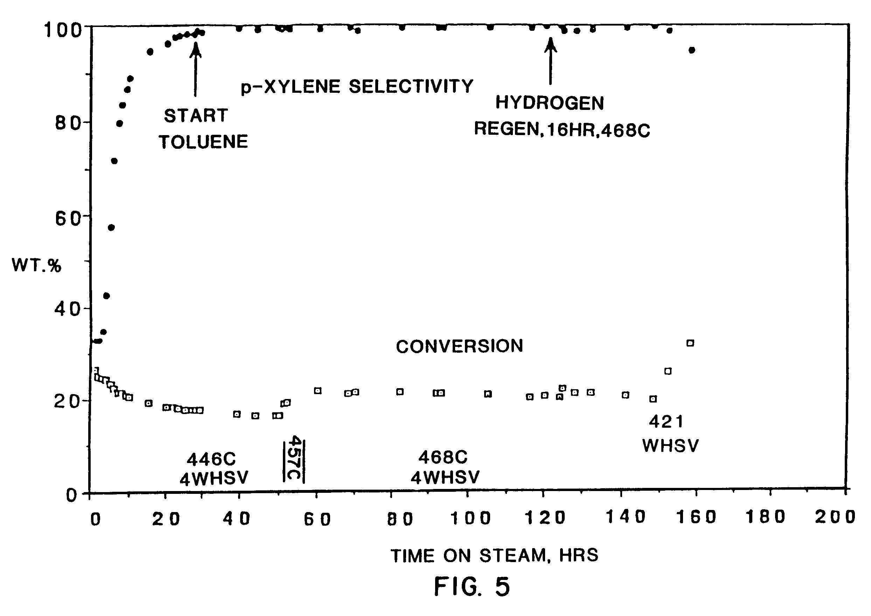

Silicone trim selectivation of the 10% Si0₂-HZSM-5 was carried out at 446°C, 3550 kPa (500 psig), 4.0 WHSV, and hydrogen/hydrocarbon ratio = 2. Table 9 and Figure 5 show toluene conversion and para-xylene selectivity for 10% Si0₂-HZSM-5 as a function of time on stream.

**TABLE 9**

| **Silicone Selectivation of 10% Si0**_{**2**}**-HZSM-5** | | |
|---|---|---|
| Time on Stream, hrs | Toluene Conversion, wt% | Para-xylene in Xylenes, wt% |
| 2 | 25 | 33 |
| 4 | 24 | 43 |
| 6 | 23 | 72 |
| 8 | 21 | 84 |
| 10 | 21 | 89 |
| 15 | 19 | 94 |
| 20 | 18 | 96 |
| 28 | 18 | 98 |

The silicone trim selectivation substantially increased para-xylene selectivity from 33% to 98% over 28 hours on stream. Feed was then changed to 100% toluene. Over the next ten hours the selectivity increased to 99% at 16% conversion. To further increase conversion, the temperature was increased to 457°C and shortly thereafter to 468°C. The conversion rose to 21%, then decreased slightly to 20% over the next 80 hours. The para-xylene selectivity increased from 99.2% to 99.6% over the same 80 hours.

Compared to the HZSM-5 of Example 1, the 10% Si0₂-HZSM-5 (pre-selectivated) catalyst of Example 25 showed a substantially higher selectivation rate. For pre-selectivated HZSM-5, 89% para-xylene selectivity was achieved after only 10 hours on stream (17 times faster than the 170 hours for the HZSM-5 parent). Also, the time needed to reach optimum para-selectivation, 1 day for pre-selectivated HZSM-5 compared to 1 week for HZSM-5, was shorter despite the higher selectivation temperature for HZSM-5(480°C vs. 446°C).

The total phenylmethyl silicone consumption was 6.80 g silicone per g HZSM-5 and 1.42 gram of silicone per gram of pre-selectivated HZSM-5. Thus trim-selectivation of the pre-selectivated HZSM-5 consumed nearly five (4.79) times less silicone than in the case of the non-pre-selectivated a catalyst.

### EXAMPLE 26

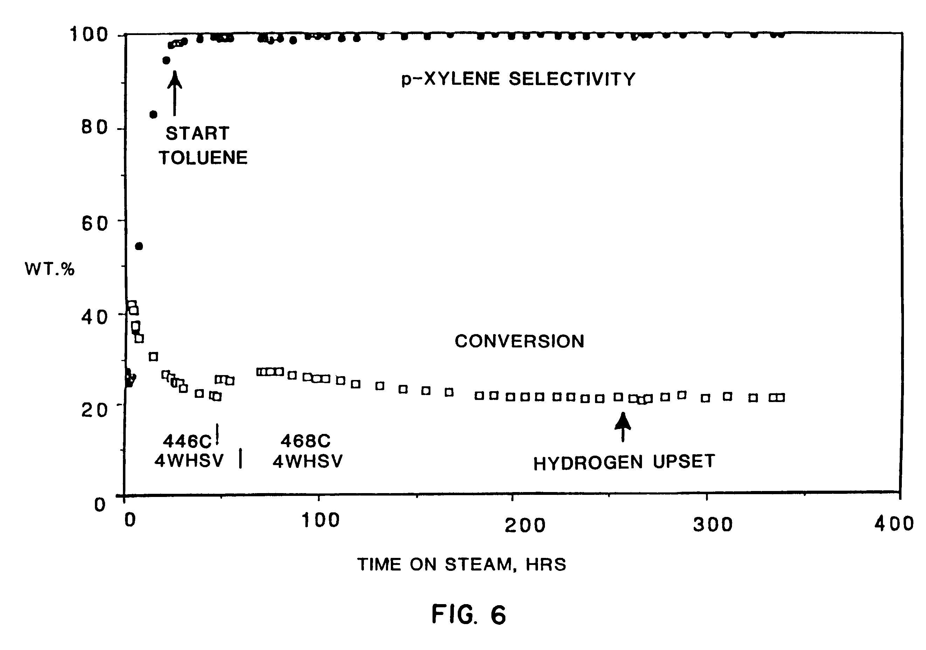

Example 25 was repeated but with the pre-selectivated catalyst containing only 5% added silica. Table 10 and Figure 6 show toluene conversion and para-xylene selectivity for 5% Si0₂-HZSM-5 as a function of time on stream.

**TABLE 10**

| **Silicone Selectivation of 5% Si0**_{**2**}**-HZSM-5** | | |
|---|---|---|
| Time on Stream, hrs | Toluene Conversion, wt% | para-xylene in Xylenes, wt% |
| 2 | 41 | 25 |
| 4 | 41 | 27 |
| 5 | 38 | 36 |
| 7 | 35 | 54 |
| 14 | 31 | 83 |
| 21 | 27 | 95 |
| 26 | 25 | 98 |

Silicone trim-selectivation substantially increased para-xylene selectivity from 25% to 98% over 26 hours on stream. Compared to 10% Si0₂-HZSM-5, the 5% Si0₂ catalyst showed consistently higher conversion over the one day selectivation time. Feed was then changed to 100% toluene. Over the next 6 hours the selectivity increased to 99% at 24% conversion, temperature was increased to 468°C and WHSV was decreased to 3. Conversion increased to 27%, then gradually decreased to and remained constant at 21% for 6 days (146 hours). Correspondingly, the para-xylene selectivity was initially unchanged at 99% then gradually increased to and remained constant at 99.6%-99.9% for 6 days when the run was arbitrarily terminated.

### EXAMPLE 27

A 0.05% Pt-10% SiO₂-HZSM-5 catalyst was prepared by adding 2.50 g of the 10% SiO₂-HZSM-5 prepared in Example 25 to 12.5 cc 1M ammonium nitrate solution. After 1.5 hours, a solution of 0.0025 g tetraamine platinum(II)nitrate in approximately 0.5 cc water was added. After standing overnight the catalyst was filtered, washed with water, and air calcined at 5°C/min to 350°C, then 3 hours at 350°C.

Toluene disproportionation was carried out over 2.00 g of the resultant catalyst at 446°C, 3550 kPa (500 psig), 4 WHSV, and a hydrogen/hydrocarbon mole ration of 2.0. Table 11 shows the product distribution compared to that of Pt-free silica-modified HZSM-5 from Example 25 tested under the same operating conditions. At similar toluene conversion, the ethylbenzene product was reduced by nearly a factor of 12 using the Pt-catalyst. The undesirable C₉⁺ aromatics product also was reduced by nearly a factor of 2.

**TABLE 11**

| Component, wt% | Pt-SiO₂-HZSM-5 | SiO₂-HZSM-5 |
|---|---|---|
| Benzene | 45.84 | 41.65 |
| Ethylbenzene | 0.05 | 0.59 |
| Xylenes | 43.12 | 55.98 |
| C₉₊ Aromatics | 0.99 | 1.78 |
| | $\overline{\text{100.00}}$ | $\overline{\text{100.00}}$ |
| Ethylbenzene in C₈, wt. | 0.10 | 1.18 |
| p-Xylene in Xylenes, wt% | 25.8 | 29.8 |
| Toluene Conversion, wt% | 35 | 34 |

### EXAMPLE 28

The catalyst of Example 27 was treated in situ with a 1% solution of phenymethylpolysiloxane in toluene at 446°C, 3550 kPa (500 psig), 4 WHSV, and a hydrogen/hydrocarbon mole ratio of 2.0. After 32 hours on stream the feed was changed to 100% toluene. Table 12 shows the product distribution compared to that of Pt-free, siloxane treated, silica-modified HZSM-5 tested under the same operating conditions.

**TABLE 12**

| Component, wt% | Pt-SiO₂-HZSM-5 | SiO₂-HZSM-5 |
|---|---|---|
| Benzene | 46.62 | 38.43 |
| Ethylbenzene | 0.33 | 1.18 |
| Xylenes | 52.35 | 58.56 |
| C₉+ Aromatics | 0.70 | 1.83 |
| | $\overline{\text{100.00}}$ | $\overline{\text{100.00}}$ |
| Ethylbenzene in C₈, wt% | 0.63 | 1.98 |
| p-Xylene in Xylenes, wt% | 98.4 | 98.7 |
| Toluene Conversion, wt% | 25 | 22 |

At similar toluene conversion, the ethylbenzene product was reduced by a factor of 3.6 using the Pt-catalyst while the p-xylene selectivities remained very high at 98.4%-98.7%. The undesirable C₉+ aromatics product was also reduced by nearly a factor of 3.

The results of Examples 29-31 which are reported in Table 13, indicate the beneficial effect on ethylbenzene in the product stream by the addition of platinum to the catalytic molecular sieve.

### EXAMPLE 29

Silicone trim-selectivation of a 10% SiO₂-HZSM-5 was carried out using 1% phenylmethyl silicone in a toluene feed at 446°C, 3550 kPa (500 psig), 4.0 WHSV, and a hydrogen/hydrocarbon ratio = 2. At 31 hours on stream the feed was changed to 100% toluene. At 52 hours on stream the temperature was increased to 468°C and at 165 hours the WHSV was lowered to 3.0. The data at 39 days on stream are shown in column 1 of Table 13.

### EXAMPLE 30

Silicone selectivation of a 0.025%Pt 10%SiO₂-HZSM-5 was carried out using 1% phenylmethyl silicone in a toluene feed at 446°C, 3550 kPa (500 psig), 4.0 WHSV, and a hydrogen/hydrocarbon ratio = 2. At 56 hours on stream the feed was changed to 100% toluene. At 73 hours on stream the temperature was increased to 468°C. The data at 7 days on stream are shown in column 2 of Table 13.

### EXAMPLE 31

Silicone selectivation of a nitric acid activated 0.05% Pt 10% SiO₂-HZSM-5 was carried out using 1% phenylmethyl silicone in a toluene feed at 446°C, 3550 kPa (500 psig), 4.0 WHSV, and a hydrogen/hydrocarbon ratio = 2. At 27 hours on stream the feed was changed to 100% toluene. Temperature, WHSV, and hydrogen/hydrocarbon ratio were varied during the run. The data at 13 days on stream are shown in column 3 of Table 13.

**TABLE 13**

| | Silicone | Silicone/Pt | |
|---|---|---|---|
| | Ex. 29 | Ex. 30 | Ex. 31 |
| Reaction Conditions | | | |
| Temperature, °C | 468 | 468 | 431 |
| Pressure, psig | 500 | 500 | 500 |
| H₂/HC | 2 | 2 | 8 |
| WHSV | 3 | 4 | 4 |
| Time on Stream, days | 39 | 7 | 13 |
| Toluene Conversion, wt% | 23 | 20 | 21 |

| Products, wt% | | | |
|---|---|---|---|
| C₅⁻ | 2.5 | 2.5 | 2.5 |
| Benzene | 43.0 | 43.6 | 47.2 |
| Ethylbenzene | 1.9 | 0.2 | 0.1 |
| Xylenes | 50.4 | 53.1 | 50.0 |
| Ethyltoluenes | 1.9 | 0.5 | 0.2 |
| C₁₀⁺ | 0.3 | 0.1 | 0.0 |
| | $\overline{\text{100.0}}$ | $\overline{\text{100.0}}$ | $\overline{\text{100.0}}$ |
| p-Xylene | 99.7 | 98.7 | 99.7 |
| m-Xylene | 0.3 | 1.3 | 0.3 |
| o-Xylene | tr. | tr. | tr. |
| | $\overline{\text{100.0}}$ | $\overline{\text{100.0}}$ | $\overline{\text{100.0}}$ |
| Benzene/Xylenes, m/m | 1.2 | 1.1 | 1.3 |
| p-XylenePurity, wt% | 97.8 | 98.3 | 99.5 |

Example 29-31 indicate that the levels of ethylbenzene in the reaction products of the present invention can be reduced by using a catalytic molecular sieve with a hydrogenation/dehydrogenation function such as platinum incorporated into the catalytic molecular sieve. The level of ethylbenzene in the product stream is preferably at a commercially acceptable level of not greater than 0.3%, and is most preferably not greater than about 0.2%.

As stated above, the present invention advantageously provides a product stream having a high para-xylene purity with respect to the other C₈ products. Table 14 provides the relative proportions of para-xylene to various combinations of other products.

**TABLE 14**

| **Comparison of Product Parameters** CATALYST | | | | |
|---|---|---|---|---|
| Parameter | Silicone | Silicone/Pt | | Calculated Equilibrium Value |
| | Ex. 29 | Ex. 30 | Ex. 31 | |
| p-Xylene/EB | 26.4 | 262 | 498 | 2.5 |
| p-Xyl/EB+m,o-xyl (other C₈) | 23.9 | 58.2 | 166 | 2.5 |
| p-Xyl/EB+m,o-Xyl+C₉ (other C₈+C₉) | 12.6 | 37.4 | 99.6 | 1.6 |
| p-Xylene purity (in all C₈s), wt% | 95.7 | 98.3 | 99.5 | 71.8 |
| p-Xylene yield (based on all products and toluene), wt% | 10.6 | 10.6 | 10.2 | 11.9 |

### EXAMPLE 32

Toluene alkylation with ethylene was carried out at 468°C and 800 kPa (100 psig) over 1.0g SiO₂-HZSM-5 catalyst trim selectivated using 1% phenylmethyl silicone in toluene at 446°C, 500 psig, 4 WHSV, and H₂/HC=2. Toluene was pumped at 4 WHSV. Ethylene and hydrogen were cofed to maintain the mole ratio toluene/ethylene/hydrogen = 8/1/3. At 10% toluene conversion (80% of the 12.5% theoretical maximum conversion), the selectivity to p-ethyltoluene was a very high 99.1% with only 0.9% m-ethyltoluene.

### EXAMPLE 33

Toluene alkylation with methanol was carried out at 448°C and 3550 kPa (500 psig) over 2.00g SiO₂-HZSM-5 catalyst trim selectivated as described above. Toluene was pumped at 4 WHSV. Methanol and hydrogen were cofed to maintain the mole ratio toluene/methanol/hydrogen = 4/1/8. At 14% toluene conversion (56% of the theoretical 25% maximum conversion), the selectivity to p-xylene was a very high 99.9% with only 0.1% m-xylene.

### EXAMPLE 34

Conversion of n-heptane was carried out 446°C, 100 kPa (0 psig), and 4 WHSV over 2.00g SiO₂-HZSM-5 catalyst trim selectivated as described above. The aromatic product distribution at 21% heptane conversion is shown in the following table. The p-xylene selectivity in the xylene fraction was a very high 99.3% with only 0.7% m-xylene.

| Component | Wt.% |
|---|---|
| Benzene | 6.2 |
| Toluene | 44.6 |
| Ethylbenzene | 4.7 |
| Xylenes | 39.5 |
| p-Ethyltoluene | 5.0 |
| | $\overline{\text{100.0}}$ |

### EXAMPLE 35

Conversion of methanol was carried out at 371°C, 100 kPa (0 psig), and 1 WHSV over 1.00g SiO₂-HZSM-5 catalyst trim selectivated as described above. The liquid product distribution at 100% methanol conversion is shown in the following table.

| Component | Wt.% |
|---|---|
| C₅-C₆ | 2.0 |
| Benzene | 7.3 |
| Toluene | 84.0 |
| Ethylbenzene | 2.2 |
| Xylenes | 42.1 |
| C₉₊ | 12.4 |
| | $\overline{\text{100.0}}$ |

### EXAMPLE 36

A Fries reaction of phenylacetate was carried out at 400°C, 800 kPa (100 psig), and 1 WHSV over SiO₂-HZSM-5 catalyst trim selectivated as described above. The 4-hydroxyaceto-phenone product is obtained in high selectivity.

### EXAMPLE 37

Propylene oligomerization was carried out at 200°C, 4200 kPa (600 psig), and 0.25 WHSV over SiO₂-HZSM-5 catalyst trim selectivated as described above. Diesel and lube products were obtained with high selectivity to the desirable straight chain hydrocarbons.

### EXAMPLE 38

Distillate dewaxing was carried out at 343°C (650°F), 2900 kPa (400 psig), and 1 WHSV over SiO₂-HZSM-5 catalyst trim selectivated as described above. The product quality was substantially improved by the selective cracking of straight chain, waxy hydrocarbons. The pour point of a heavy gas oil improved from 35°C (95°F) to -7°C (20°F).

## Claims

1. A process for producing a shape selective conversion catalyst comprising converting toluene to xylenes by contacting toluene with a catalyst comprising a molecular sieve having a constraint index of 1-30 at a temperature of 100-600°C, a pressure of 100 to 14000 kPa (atmospheric to 2000 psig), a WHSV of 0.1 to 100, and a hydrogen to hydrocarbon molar ratio of 0 to 10, characterized in that the toluene is fed in the form of a vapour phase mixture which comprises at least 80% by weight of toluene and at least 0.1% by weight of a selectivating agent.

2. A process according to claim 1, wherein the molecular sieve is treated with a silicon-containing selectivating agent prior to treatment with said mixture.

3. A process according to claim 1 or claim 2, wherein the selectivating agent comprises an organosilicon compound.

4. A process according to claim 3, wherein the organosilicon compound is selected from polysiloxanes, siloxanes, silanes, disilanes, and alkoxysilanes.

5. The process according to any one of the preceding claims, wherein said molecular sieve has a constraint index of 1 to 20.

6. The process according to any one of the preceding claims, wherein said molecular sieve comprises ZSM-5.

## Patentansprüche

1. Verfahren zur Herstellung eines Katalysators für die formselektive Umwandlung, das die Umwandlung von Toluol in Xylole durch den Kontakt von Toluol mit einem Katalysator, der ein Molekularsieb mit einem Zwangsindex von 1 bis 30 umfaßt, bei einer Temperatur von 100 bis 600°C, einem Druck von 100 bis 14.000 kPa (Atmosphärendruck bis 2.000 psig), einer WHSV von 0,1 bis 100 und einem Wasserstoff/Kohlenwasserstoff-Molverhältnis von 0 bis 10 umfaßt, dadurch gekennzeichnet, daß Toluol in Form einer Mischung in der Dampfphase zugeführt wird, die mindestens 80 Gew.-% Toluol und mindestens 0,1 Gew.-% eines Selektivierungsmittels enthält.

2. Verfahren nach Anspruch 1, wobei das Molekularsieb vor der Behandlung mit der Mischung mit einem siliciumhaltigen Selektivierungsmittel behandelt wird.

3. Verfahren nach Anspruch 1 oder Anspruch 2, wobei das Selektivierungsmittel eine Organosiliciumverbindung umfaßt.

4. Verfahren nach Anspruch 3, wobei die Organosiliciumverbindung aus Polysiloxanen, Siloxanen, Silanen, Disilanen und Alkoxysilanen ausgewählt ist.

5. Verfahren nach einem der vorstehenden Ansprüche, wobei das Molekularsieb einen Zwangsindex von 1 bis 20 hat.

6. Verfahren nach einem der vorstehenden Ansprüche, wobei das Molekularsieb ZSM-5 umfaßt.

## Revendications

1. Procédé de production d'un catalyseur de conversion a sélectivité de forme comprenant la conversion du toluène en xylènes par mise en contact du toluène avec un catalyseur comprenant un tamis moléculaire ayant un indice de contrainte de 1 à 30, à une température de 100 à 600°C, une pression de 100 à 14000 kPa (pression atmosphérique à 2000 psig), une WHSV de 0,1 à 100 et un rapport molaire hydrogène/hydrocarbure de 0 à 10, caractérisé en ce que le toluène est chargé sous la forme d'un mélange en phase vapeur qui comprend au moins 80% en poids de toluène et au moins 0,1% en poids d'un agent augmentant la sélectivité.

2. Un procédé suivant la revendication 1, dans lequel le tamis moléculaire est traité avec un agent augmentant la sélectivité contenant du silicium avant d'être traité avec ce mélange.

3. Un procédé suivant les revendications 1 ou 2, dans lequel l'agent augmentant la sélectivité comprend un composé organique du silicium.

4. Un procédé suivant la revendication 3, dans lequel le composé organique du silicium est choisi parmi les polysiloxanes, les siloxanes, les silanes, les disilanes et les alcoxysilanes.

5. Un procédé suivant l'une quelconque des revendications précédentes, dans lequel le tamis moléculaire a un indice de contrainte de 1 à 20.

6. Un procédé suivant l'une quelconque des revendications précédentes, dans lequel le tamis moléculaire comprend une ZSM-5.
